Europäisches Patentamt

European Patent Office

Office européen des brevets

(11) Publication number: **0 044 134**
**B1**

## EUROPEAN PATENT SPECIFICATION

(45) Date of publication of patent specification: **05.06.85**

(21) Application number: **81302561.6**

(22) Date of filing: **09.06.81**

(51) Int. Cl.⁴: **A 61 K 9/06, A 61 K 31/41,**
**A 61 K 31/18, C 07 D 285/12**

(54) Composition for reducing intraocular pressure.

(30) Priority: **09.07.80 US 167220**

(43) Date of publication of application:
**20.01.82 Bulletin 82/03**

(45) Publication of the grant of the patent:
**05.06.85 Bulletin 85/23**

(84) Designated Contracting States:
**AT BE CH DE FR GB IT LI LU NL SE**

(56) References cited:
**FR-A-2 332 008**
**FR-A-2 468 366**

**CHEMICAL ABSTRACTS, vol. 87, 1977, page 97, abstract 127621e Columbus, Ohio, US T.H. MAREN et al. "The pharmacology of methazolamide in relatin to the treatment of glaucoma"**

The file contains technical information submitted after the application was filed and not included in this specification

(73) Proprietor: **UNIVERSITY OF FLORIDA**
**207 Tigert Hall**
**Gainesville Florida 32611 (US)**

(72) Inventor: **Maren, Thomas H.**
**2306 Southwest 13th Street Apartment No. 806**
**Gainesville Florida 32608 (US)**

(74) Representative: **Gold, Tibor Zoltán**
**T.Z.GOLD & COMPANY 9, Staple Inn**
**London WC1V 7QH (GB)**

(56) References cited:
**CHEMICAL ABSTRACTS, vol. 80, 1974, page 126, abstract 79635u Columbus, Ohio, US M. KANDEL et al. "Reaction of bromoacetazolamide with crystals of human carbonic anhydrase C"**
**CHEMICAL ABSTRACTS, vol. 91, 1979, page 58, abstract 13809z, Columbus, Ohio, US K.N. GELATT et al. "Ocular hypotensive effects of carbonic anhydrase inhibitors in normotensive and glaucomatous beagles"**
**JOURNAL OF THE AMERICAN PHARMACEUTICAL ASSOCIATION, vol. 19, no. 8, August 1958; pages 474-76; no. 11, November 1958, p. 665-66**
**Biochem, Biophys. Res. Comms. vol. 56 (3), 1974, pp. 568-574**

Courier Press, Leamington Spa, England.

## Description

### Technical field

This invention is directed to a process and composition for reducing intraocular pressure and reducing aqueous humor formation by applying topically to the cornea an effective amount of an aqueous solution of a carbonic anhydrase inhibitor.

### Background of the invention

Glaucoma is well known as a condition in which the internal pressure in the eye increases to the extent that it causes damage to the optic nerve and may eventually cause blindness. This condition is primarily caused by the failure of aqueous humor to properly drain from the eye, resulting in a high internal or intraocular pressure. It has been recognized that the formation of aqueous humor is the result of the activity of the enzyme carbonic anhydrase which is employed by the human body to reversibly catalyze the hydration of carbon dioxide. Compounds, principally heterocyclic sulfonamides, are known which inhibit the activity of carbonic anhydrase and thus control the production of aqueous humor and the intraocular pressure resulting therefrom. [Havener, Ocular Pharmacology, 4th Ed. (1978, C.V. Moseby); Maren, Investigative Ophthalmology, Vol. 13, pp479—484 (1974); Becker, Am. J. of Ophthalmology, *39*, 177 (1955)].

It is necessary to administer these materials parenterally in order to achieve intraocular pressure reduction. Parenteral administration requires relatively large dosages and very often results in the patient experiencing fatigue, depression, anorexia, numbness and tingling sensations.

It has now been discovered that certain carbonic anhydrase inhibitors can be administered topically, i.e., applied directly to the cornea, and that these compounds will penetrate the cornea and be immediately effective in inhibiting the activity of carbonic anhydrase and decreasing intraocular pressure and aqueous humor flow resulting from this activity.

The topical or local applicability of intraocular pressure depressants offers several major advantages over drugs requiring parenteral administration. Topical applicability avoids the unpleasant side effects described above which result from systemic applications of carbonic anhydrase inhibitors. In addition, topical application enables a more rapid, localized concentration of the drug at the situs requiring the drug's action.

The use of acetazolamide in treating glaucoma is known, e.g. from French Patent Specification No. 2,468,366. However, if it reduces intraocular pressure at all, it does so only after prolonged treatment that makes it an impracticable therapy.

Bromoacetazolamide is known to react *in vitro* with carbonic anhydrase (M. Kendal *et al*, Biochemical and Biphysical Research Communication Vol. 56, No. 3, 1974, pages 568—574) but it has not hitherto been known whether it is any more effective in treating glaucoma than acetazolamide.

### Brief summary of the invention

This invention provides a composition particularly adapted for reducing intraocular pressure and reducing aqueous humor formation. It has been discovered that the topical application to the cornea of certain anhydrase inhibitors effects a reduction of aqueous humor formation and intraocular pressure. These carbonic anhydrase inhibitors comprise pahrmaceutically acceptable sulfonamides having the following properties:

    a. a pKa of not greater than 7.0 or a sufficient solubility in water to form a 1—5% by weight solution;

    b. ether partition coefficient of at least 1.0;

    c. chloroform partition coefficient of at least 0.01;

    d. dissociation constant against carbonic anhydrase of not more than $3 \times 10^{-8}$ molar;

    e. first order rate constant for penetration of said sulfonamide through an isolated rabbit cornea of at least $0.0002h^{-1}$.

    f. not injurious to the cornea; and

    g. stable in solution and in contact with the cornea.

### Detailed description of the invention

In accordance with the invention it has been found that certain carbonic anhydrase inhibitors can be safely and advantageously applied directly to the cornea in the form of drops of aqueous solution. These inhibitors must have certain properties in order to effectively function in this fashion. First, the inhibitor in its acid form must have a pKa which is not greater than 7.0, or the inhibitor in the acid form must be sufficiently soluble in water to produce a 1—5% solution. This property is important in that it permits the compound to be used in an 1—5% aqueous solution at a pH below 8 and therefore can be applied to the eye at a relatively neutral pH. The pKa may be measured by titrating the compound with NaOH and finding the point at which half of the compound is neutralized. The pH at this point is the pKa.

Another necessary property of these compounds is that they must possess an ether partition coefficient of at least 1.0. This property in conjunction with the chloroform coefficient is a measure of the lipid solubility of this compound. This is a critical feature since the compound must be readily absorbed by the lipid materials, in the eye and be available for intimate contact with carbonic anhydrase so as to control its activity. This property is measured by preparing an aqueous solution of the compound at pH7 and shaking the solution with an equal volume of ether until equilibrium is achieved in the system. An ether layer and an aqueous layer are formed, separated, and each analyzed for its content of the compound. The coefficient is the ratio of the amount of the compound in the ether layer to the amount of the compound in the aqueous layer.

See, e.g., Maren, J., Pharm. Expt. Therap. *130*, p 26, (1960) for the method of analysis for the compound in each layer.

Another critical property necessary in the inhibitors employed in this invention is the chloroform partition coefficient which must be at least 0.01. This property also relates to the lipid solubility of the compound employed and it is measured as described above with respect to ether. The procedure is the same as that described above except that chloroform is employed in place of ether.

Another necessary property in the inhibitors employed in this invention is that they must have a dissociation constant against carbonic anhydrase of not more than $3 \times 10^{-8}$ molar. This property denotes that the compound has high activity against carbonic anhydrase in the ciliary process of the eye which is, at least in part, responsible for the secretion of aqueous humor. The dissociation constant in the concentration of the compound that will inhibit one-half of the carbonic anhydrase in a test system wherein the conditions are so arranged that the compound is present in excess of the carbonic anhydrase. The test system is described in Maren, *supra*, and in a subsequent article by Maren in the same volume at page 389.

Still another critical property of the inhibitor compounds employed in this invention is that the compound must have a first order rate constant for penetration of the compound through the isolated cornea of a rabbit of at least $0.0002 h^{-1}$. This property is important in that it sets a standard for a speed in which the inhibitor will pass through the cornea to the interior of the eye and be available to inhibit the activity of carbonic anhydrase in that location. This rate constant is measured by employing a cornea which has been excised from an animal and mounted in a laboratory apparatus in such a way that two different aqueous solutions are separated from each other by that cornea and analysis can be made of the solution at timed intervals so as to determine the rate at which the dissolved compound in one solution appears in the second solution. Analysis of the solutions may be made by the method of the Maren articles cited above. The permeability system employing an animal cornea is described in Hull et al, Invest. Ophthal. *13*, p 457, 1974.

Still another critical property of the compounds employed in this invention is that they be pharmaceutically acceptable and not be injurious to the cornea being treated.

The final property which is necessary for a compound used in this invention is that it be stable against decomposition in solution and in contact with the cornea. This property may be tested by dissolving the compound in aqueous solution and in solution containing corneal tissue to determine whether the compound has remained stable or has decomposed into other materials.

None of the carbonic anhydrase inhibitors previously employed by parenteral administration is useful because they do not meet all of the criteria mentioned above and therefore cannot be used topically. Perhaps the best known inhibitor in the prior art is acetazolamide (2-acetylamino-1,3,4-thiadiazole-5-Sulfonamide) which cannot be made into a water solution below pH8 at concentration greater than 0.05%. The pKa is 7.4; the ether partition coefficient is 0.14 and the chloroform coefficient is 0.001. This material has been tried and found to be completely unsuitable in lowering intraocular pressure by topical administration. [Foss, Am. J. Ophthalmology, 39, 336 (1955)]. Methazolamide (2 - acetylimino - 3 - methyl - $\Delta^2$ - 1,3,4 - thiadiazoline - 5 - Sulfonamide) is another known carbonic anhydrase inhibitor but it is unsuitable in the process of this invention. This compound has a pKa of 7.4 and cannot be made into a neutral solution with a concentration of more than about 0.2%. In a series of tests the intraocular pressure was not reduced and the production of aqueous humor was not reduced except to a very slight extent in one of the series of tests. Still another prior art inhibitor is ethoxzolamide (6 - ethoxy - benzothiazole - 2 - sulfonamide) having properties somewhat similar to those of methazolamide. This material has a pKa of 8.1 and cannot be made into an aqueous solution at a concentration above about 0.004%. This drug is ineffective in topically treating the eye to reduce intraocular pressure.

An inhibitor which is useful in this invention is 2 - bromoacetylamino - 1,3,4 - thiadiazole - 5 - sulfonamide, commonly named bromacetazolamide. This compound has a pKa of 5.7; an ether partition coefficient of 2.0; a chloroform partition coefficient of 0.01; a dissociation constant of $10^{-8}$ molar; a rabbit cornea penetration rate constant of $0.003 h^{-1}$; it produces no injury or clouding in the cornea upon being applied in a 2—5% solution; and it is stable in solution for at least several weeks. The preferred concentration in which this drug may be applied to the eye is 1—5%, the actual choice being dependent upon several factors such as, the severity of the disease and individual patient variations.

Another compound which is useful in the process of this invention is 2 - trifluoroacetylimino - 3 - methyl - $\Delta^2$ - 1,3,4 - thiadiazoline - 5 - sulfonamide, its common name being trifluormethazolamide. This compound meets all of the qualifications as set forth for the composition of this invention, although it is not as stable in solution as bromacetazolamide. This compound has a pKa of 6.4; an ether partition coefficient of 6.0; a chloroform partition coefficient of 0.3; a dissociation constant of $3 \times 10^{-8}$ molar; a rabbit cornea penetration rate constant of $0.003 h^{-1}$; it does not injure or cloud the cornea in a 2—5% solution; and is stable in solution for two hours.

Still another compound useful in this invention is 2 - trichloroacetylimino - 3 - methyl - $\Delta^2$ - 1,3,4 - thiadiazoline - 5 - sulfonamide, its common name being trichlormethazolamide. It has a pKa of 6.8; an ether partition coefficient of 21, a chloroform partition coefficient of 3.0; a dissociation constant of $2 \times 10^{-8}$ molar; a rabbit cornea penetration rate constant of greater than $0.001 \ h^{-1}$; it does not injure or cloud the cornea in 2—5% solution; and it is stable in solution for at least six hours.

All three of the above-described compounds, i.e. bromacetazolamide, trifluormethazolamide, and trichlormethazolamide as well as other compounds meeting the above-listed criteria are useful and operable in lowering intraocular pressure and in reducing the formation of aqueous humor by topical treatment of the eye in accordance with this invention.

Sulfonamides which are sufficiently soluble in water to form 1—5%, by weight, solution may be utilized in the acid form. Those sulfonamides that are not sufficiently water soluble are utilized in the form of their pharmaceutically acceptable water-soluble salts, i.e., sodium, potassium, triethanolamine, etc. The sulfonamide solutions are applied topically to the eye by exposing the entire cornea to the solution for a time sufficient for penetration into the eye of an amount of carbonic anhydrase inhibitor sufficient to effect a reduction in aqueous humor formation and intraocular pressure. Generally, when employing solutions containing 1—5% by weight of sulfonamide, exposure to the cornea for from about 10 to 30 minutes is adequate to enable an effective penetration of sulfonamide. The exact time of exposure will vary depending on the nature of the sulfonamide.

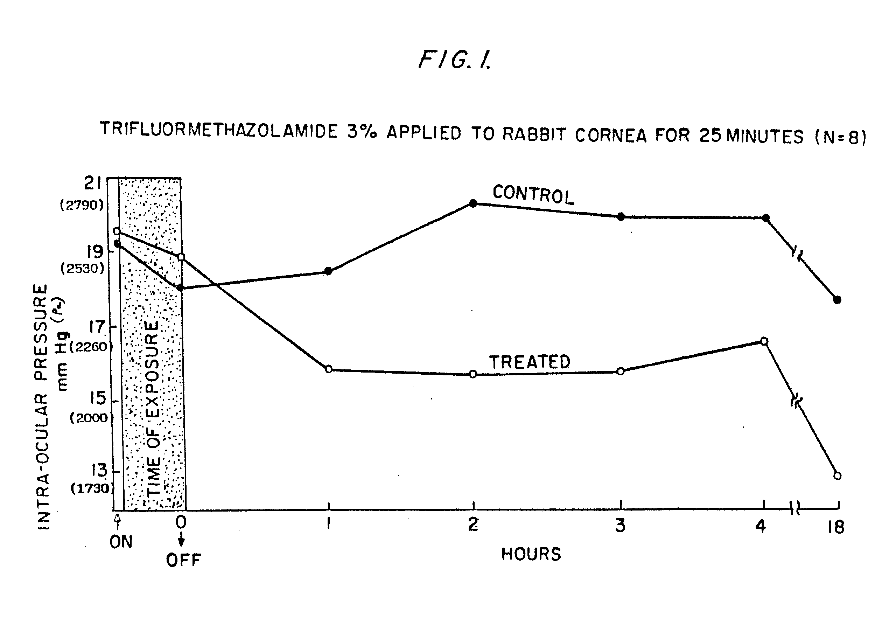

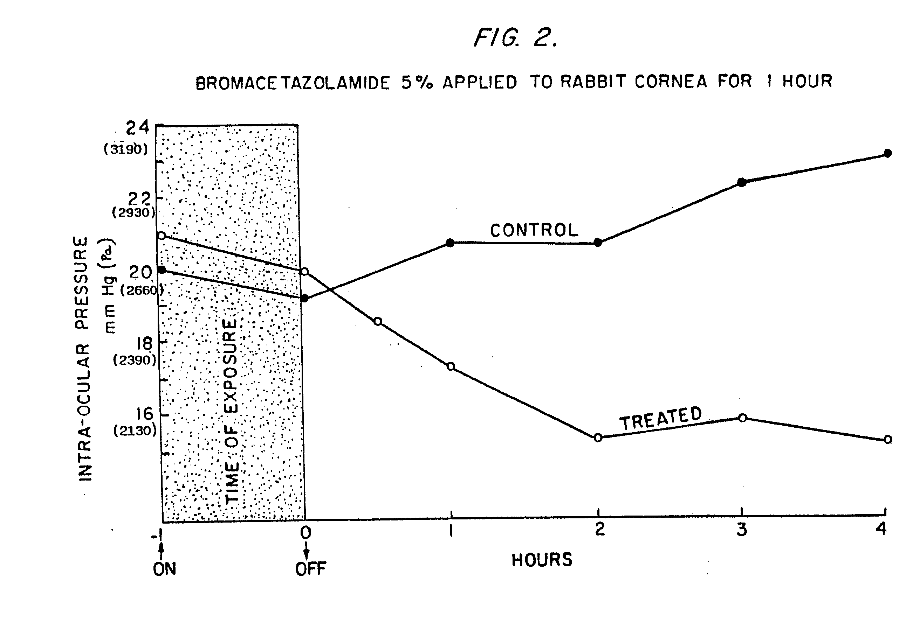

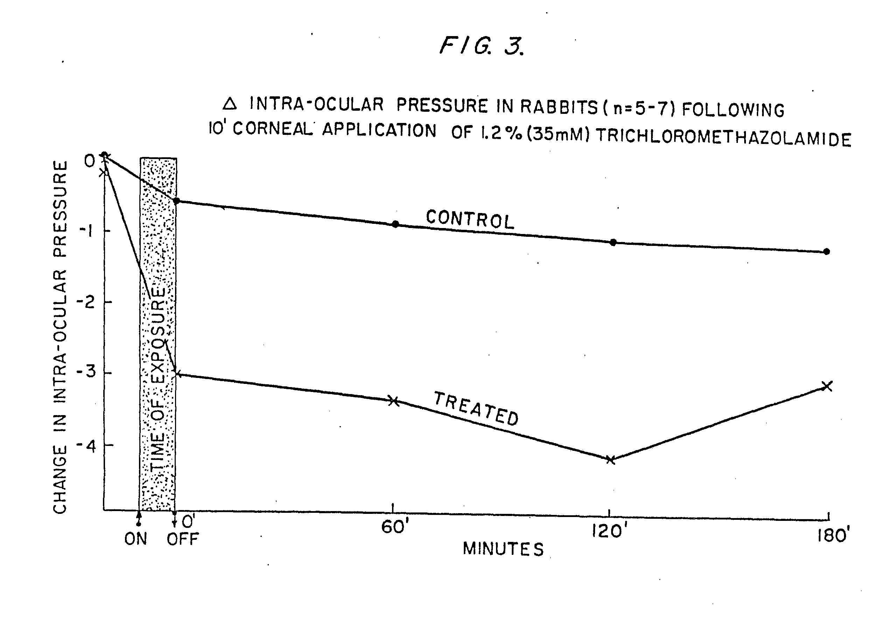

Figs. 1—3 demonstrate the effect on the intraocular pressure in the eye of rabbits exposed to solutions of sulfonamides according to the invention. The shaded portions of the figures indicate the times of exposure of the eye to the active solutions. The other eye of the rabbit is used as a control. Intraocular pressure is measured utilizing the pneumotonograph according to the method of Quigly et al (Amer. J. Ophthal. *80,* p 266 (1975). Briefly, an electronically controlled probe is gently placed on the cornea. The probe consists of a membrane, piston, and gas chamber arranged so that the eye pressure is translated electronically to a scribe on moving graph paper which records the pressure.

The volume and flow of aqueous humor is measured according to the methods of Oppelt, Invest. Opthal., Vol. 6, p. 76 (1967).

**Claims for the Contracting States BE CH DE FR GB IT LI LU NL SE**

1. A composition adapted for topical application to the eye in unit dosage form comprising an aqueous solution of an amount of a carbonic anhydrase inhibitor sufficient to reduce aqueous humor formation and to reduce intraocular pressure, the inhibitor comprising a pharmaceutically acceptable sulfonamide having the following properties:

(a) a pKa of not greater than 7.0 or a sufficient solubility in water to form a 1—5%, by weight, solution;

(b) ether partition coefficient of at least 1.0;

(c) chloroform partition coefficient of at least 0.01;

(d) dissociation constant against carbonic anhydrase of not more than $3 \times 10^{-8}$ molar;

(e) first order rate constant for penetration of said sulfonamide through a rabbit cornea of at least $0.0002 \ h^{-1}$;

(f) not injurious to the cornea; and

(d) stable in solution and in contact with the cornea.

2. A composition as claimed in claim 1, wherein said aqueous solution contains 1 to 5% by weight of said carbonic anhydrase inhibitor.

3. The composition according to claim 1 or 2, wherein said sulfonamide is a heterocyclic sulfonamide.

4. The composition according to claim 1 or claim 2, wherein said sulfonamide is a thiadiazoline sulfonamide.

5. The composition according to claim 4, wherein said sulfonamide is 2 - trifluoroacetylimino - 3 - methyl - $\Delta^2$ - 1,3,4 - thiadiazoline - 5 - sulfonamide.

6. The composition according to claim 4, wherein said sulfonamide is 2 - trichloroacetylimino - 3 - methyl - $\Delta^2$ - 1,3,4 - thiadiazoline - 5 - sulfonamide.

7. The composition according to claim 3, wherein said sulfonamide is 2 - bromoacetylamino - 1,3,4 - thiadiazole - 5 - sulfonamide.

8. The compound 2 - trifluoro - acetylimino - 3 - methyl - $\Delta^2$ - 1,3,4 - thiadiazoline - 5 - sulfonamide.

9. The compound 2 - trichloroacetylimino - 3 - methyl - $\Delta^2$ - 1,3,4 - thiadiazoline - 5 - sulfonamide.

**Claims for the Contracting State AT**

1. A process for making a composition adapted for topical application to the eye in the unit dosage form, which process comprises dissolving in an aqueous solvent an amount of a carbonic anhydrase inhibitor that is sufficient to reduce aqueous humor formation and to reduce intraocular pressure, the inhibitor comprising a pharmaceutically acceptable sulfonamide having the following properties:

a) a pKa of not greater than 7.0 or a sufficient solubility in water to form a 1—5%, by weight, solution;

b) ether partition coefficient of at least 1.0;

c) chloroform partition coefficient of at least 0.01;

d) dissociation constant against carbonic anhydrase of not more than $3 \times 10^{-8}$ molar;

e) first order rate constant for penetration of said sulfonamide through a rabbit cornea of at least $0.0002 \ h^{-1}$;

f) not injurious to the cornea; and

g) stable in solution and in contact with the cornea.

2. A process as claimed in claim 1, wherein the amount of the inhibitor is 1 to 5% by weight.

3. A process according to claim 1 or claim 2, wherein said sulfonamide is a heterocyclic sulfonamide.

4. A process according to claim 3, wherein said sulfonamide is a thiadiazoline sulfonamide.

5. A process according to claim 3, wherein said sulfonamide is 2-bromoacetylamino - 1,3,4 - thiadiazole - 5 - sulfonamide.

6. A process according to claim 4, wherein said sulfonamide is 2 - trifluoroacetylimino - 3 - methyl - $\Delta^2$ - 1,3,4 - thiadiazoline - 5 - sulfonamide.

7. A process according to claim 4, wherein said sulfonamide is 2 - trichloroacetylimino - 3 - methyl - $\Delta^2$ - 1,3,4 - thiadiazoline - 5 - sulfonamide.

**Patentansprüche für die Vertragsstaaten: BE CH, DE, FR, GB, IT, LI, LU, NL SE**

1. Zusammensetzung zur lokalen Anwendung am Auge in Form von Einheitsdosen mit einer für die Verringerung der Bildung von Kammerwasser und zur Verringerung des Augeninnendruckes ausreichenden Menge eines Karboanhydraseinhibitors in einer wässerigen Lösung, welcher ein pharmazeutisch anwendbares Sulfonamid mit den folgenden Eigenschaften aufweist:

a) einen pKa-Wert von nicht größer als 7,0 oder eine ausreichende Löslichkeit in Wasser zur Bildung einer Lössung von 1—5 gew.%,

b) einen Äther-Verteilungskoeffizienten von wenigstens 1,0,

c) einen Chloroform-Verteilungskoeffizienten von wenigstens 0,01,

d) eine Dissoziationskonstante gegen Karboanhydrase von nicht mehr als $3\times10^{-8}$ molar,

e) eine Geschwindigkeitskonstante erste Ordnung für das Hindurchdringen des Sulfonamides durch eine Kaninchen-Hornhaut von wenigstens 0.0002,

f) nicht schädlich für die Hornhaut und

g) stabil in der Lösung und im Kontakt mit der Hornhaut.

2. Zusammensetzung nach Anspruch 1, bei welcher die wässrige Lösung 1—5 gew.% des Karboanhydraseinhibitors enthält.

3. Zusammensetzung nach Anspruch 1 oder 2, bei welcher das Sulfonamid ein heterozyklisches Sulfonamid ist.

4. Zusammensetzung nach Anspruch 1 oder 2, bei welcher das Sulfonamid ein Thiadiazolin-Sulfonamid ist.

5. Zusammensetzung nach Anspruch 4, bei welcher das Sulfonamid 2 - Trifuorazetylimino - 3 - methyl - $\Delta^2$ - 1,3,4 - thiadiazolin - 5 - sulfonamid ist.

6. Zusammensetzung nach Anspruch 4, bei welcher das Sulfonamid 2 - Trichlorazetylimino - 3 - methyl - $\Delta^2$ - 1,3,4 - thiadiazolin - 5 - sulfonamid ist.

7. Zusammensetzung nach Anspruch 3, bei welcher das Sulfonamid 2 - Bromazetylamino - 1,3,4 - thiadiazol - 5 - sulfonamid ist.

8. Die Verbindung 2-Trifluor-azetylimino - 3 - methyl - $\Delta^2$ - 1,3,4 - thiadiazolin - 5 - sulfon-amid.

9. Die Verbindung 2 - Trichlorazetylimino - 3 - methyl - $\Delta^2$ - 1,3,4 - thiadiazolin - 5 - sulfon-amid.

**Patentansprüche für den Vertragsstaat: AT**

1. Verfahren zum Herstellen einer Zusammensetzung zur lokalen Anwendung am Auge in Form von Einheitsdosen, mit welchem eine für die Verringerung der Bildung von Kammerwasser und zur Verringerung des Augeninnendruckes ausreichenden Menge eines Karboanhydraseinhibitors in einem wässrigen Lösungsmittel gelöst wird, welcher ein pharmazeutisch anwendbares Sulfonamid mit den folgenden Eigenschaften aufweist:

a) einen pKa-Wert von nicht größer als 7,0 oder eine ausreichende Löslickeit in Wasser zur Bildung einer Lösung von 1—5 gew.%,

b) einen Äther-Verteilungskoeffizienten von wenigstens 1,0,

c) einen Chloroform-Verteilungkoeffizienten von wenigstens 0,01,

d) eine Dissoziationskonstante gegen Karboanhydrase von nicht mehr als $3\times10^{-8}$ molar,

e) eine Geschwindigkeitskonstante erste Ordnung für das Hindurchdringen des Sulfonamides durch eine Kaninchen-Hornhaut von wenigstens 0.0002,

f) nich schädlich für die Hornhaut und

g) stabil in der Lösung und im Kontakt mit der Hornhaut.

2. Verfahren nach Anspruch 1, bei welcher die Menge des Inhibitors 1—5 Gew.% ist.

3. Verfahren nach Anspruch 1 oder 2, bei welcher das Sulfonamid ein heterozyklisches Sulfonamid ist.

4. Verfahren nach Anspruch 3, bei welcher das Sulfonamid ein Thiadiazolin-Sulfonamid ist.

5. Verfahren nach Anspruch 4, bei welcher das Sulfonamid 2 - Bromazetylamino - 1,3,4 - Thiadiazol - 5 - Sulfonamid ist.

6. Verfahren nach Anspruch 4, bei welcher das Sulfonamid 2 - Trichlorazetylimino - 3 - methyl - $\Delta^2$ - 1,3,4 - thiadiazolin - 5 - sulfonamid ist.

7. Verfahren nach Anspruch 4, bei welcher das Sulfonamid 2 - trifluorazetylimino - 3 - methyl - $\Delta^2$ - 1,3,4 - thiadiazolin 5 - sulfonamid ist.

**Revendications pour les Etats Contractants: BE, CH, DE, FR, GB, IT, LI, LU, NL, SE**

1. Composition adaptée à l'application topique à l'oeil, en unité dosée comprenant une solution aqueuse d'une quantité d'inhibiteur de l'anhydrase carbonique suffisante pour réduire la formation de l'humeur aqueuse et réduire la pression intra-oculaire, l'inhibiteur étant formé d'un sulfonamide pharmaceutiquement acceptable ayant les propriétés suivantes:

(a) un pKa n'excèdant pas 7,0 ou une hydro-solubilité suffisante pour former une solution à 1—5% en poids;

(b) un coefficient de séparation dans l'éther d'au moins 1,0;

(c) un coefficient de séparation dans le chloroforme d'au moins 0,01;

(d) une constante de dissociation à l'égard de l'anhydrase carbonique n'excèdant pas $3 \times 10^{-8}$ molaire;

(e) une constante de vitesse d'ordre premier pour la pénétration dudit sulfonamide au travers d'une cornée de lapin d'au moins 0,0002 $h^{-1}$;

(f) une inocuité vis-à-vis de la cornée; et

(g) une stabilité en solution et au contact avec la cornée.

2. Composition selon la revendication 1, caractérisée en ce que la solution aqueuse renferme de 1 à 5% en poids dudit inhibiteur de l'anhydrase carbonique.

3. Composition selon la revendication 1 ou 2, caractérisée en ce que ledit sulfonamide est un sulfonamide hétérocyclique.

4. Composition selon la revendication 1 ou 2, caractérisée en ce que ledit sulfonamide est un thiadiazoline-sulfonamide.

5. Composition selon la revendication 4, caractérisée en ce que ledit sulfonamide est le trifluoroacétylimino - 2 - méthyl - 3 thiadiazoline - $\Delta^2$ - 1,3,4 sulfonamide - 5.

6. Composition selon la revendication 4, caractérisée en ce que ledit sulfonamide est le trichloroacétylimino - 2 - méthyl - 3 thiadiazo-line - $\Delta^2$ - 1,3,4 sulfonamide - 5.

7. Composition selon la revendication 3, caractérisée en ce que ledit sulfonamide et le bromo-acétylamino - 2 thiadiazole - 1,3,4 sulfonamide-5.

8. Trifluoroacétylimino-2 méthyl-3 thiadiazoline - $\Delta^2$ - 1,3,4 sulfonamide-5.

9. Trichloroacétylimino-2 méthyl-3 thiadiazoline - $\Delta^2$ - 1,3,4 sulfonamide-5.

### Revendications pour l'Etat Contractant: AT

1. Procédé pour la fabrication d'un composition adaptée a l'application topique à l'oeil, en unité dosée, comprenant faisant dissoudre dans une solution aqueuse une quantité d'inhibiteur de l'anhydrase carbonique suffisante pour réduire la formation de l'humeur aqueuse et réduire la pression intra-oculaire, l'inhibiteur étant formé d'un sulfonamide pharmaceutiquement acceptable ayant les propriétés suivantes:

a) un pKa n'excèdant pas 7,0 ou une hydro-solubilité suffisante pour former une solution à 1—5% en poids;

(b) un coefficient de séparation dans l'éther d'au moins 1,0;

(c) un coefficient de séparation dans le chloroforme d'au moins 0,01;

(d) une constante de dissociation à l'égard de l'anhydrase carbonique n'excèdent pas $3 \times 10^{-8}$ molaire;

(e) une constant de vitesse d'ordre premier pour la pénétration dudit sulfonamide au travers l'une cornée de lapin d'au moins 0,0002 $h^{-1}$;

(f) une inocuité vis-à-vis de la cornée; et

(g) une stabilité en solution et au contact avec la cornée.

2. Procédé selon la revendication 1, caractérisée en ce que la solution aqueuse renferme de 1 à 5% en poids dudit inhibiteur de l'anhydrase carbonique.

3. Procédé selon la revendication 1 ou 2, caractérisée en ce que ledit sulfonamide est un sulfonamide heterocyclique.

4. Procédé selon la revendication 3, caractérisée en ce que ledit sulfonamide est un thiadiazoline-sulfonamide.

5. Procédé selon la revendication 3, caractérisée en ce que ledit sulfonamide est le bromo-acétylamino-2-thiadiazole 1,3,4 sulfonamide-5.

6. Procédé selon la revendication 4, caractérisée en ce que ledit sulfonamide est le trifluoroacétylimino - 2 - méthyl - 3 thiadiazoline - $\Delta^2$ - 1,3,4 sulfonamide-5.

7. Procédé selon la revendication 4, caractérisée en ce que ledit sulfonamide et le tri-chloroacétylimino-2 méthyl-3 thiadiazo-line-$\Delta^2$-1,3,4 sulfonamide-5.

FIG. I.

TRIFLUORMETHAZOLAMIDE 3% APPLIED TO RABBIT CORNEA FOR 25 MINUTES (N=8)

*FIG. 2.*

BROMACETAZOLAMIDE 5% APPLIED TO RABBIT CORNEA FOR 1 HOUR

0 044 134

FIG. 3.

△ INTRA-OCULAR PRESSURE IN RABBITS (n=5-7) FOLLOWING
10' CORNEAL APPLICATION OF 1.2% (35mM) TRICHLOROMETHAZOLAMIDE

CONTROL

TREATED

CHANGE IN INTRA-OCULAR PRESSURE

TIME OF EXPOSURE

ON   OFF

60'        120'        180'

MINUTES

0 044 134